Europäisches Patentamt

European Patent Office

Office européen des brevets

(19)

(11) Veröffentlichungsnummer: **0 219 781**
A2

(12) # EUROPÄISCHE PATENTANMELDUNG

(21) Anmeldenummer: 86114095.2

(22) Anmeldetag: 11.10.86

(51) Int. Cl.⁴: **C 12 P 21/02**
C 07 K 15/26, C 12 N 15/00
A 61 K 45/02

(30) Priorität: 17.10.85 DE 3536939

(43) Veröffentlichungstag der Anmeldung:
29.04.87 Patentblatt 87/18

(84) Benannte Vertragsstaaten:
AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Engels, Joachim, Prof. Dr.
Feldbergstrasse 1
D-6242 Kronberg/Taunus(DE)

(72) Erfinder: Leineweber, Michael, Dr.
Heimchenweg 74
D-6230 Frankfurt am Main 80(DE)

(72) Erfinder: Seipke, Gerhard, Dr.
Brunnenweg 4
D-6200 Wiesbaden(DE)

(72) Erfinder: Uhlmann, Eugen, Dr.
Zum Talblick 31
D-6246 Glashütten/Taunus(DE)

(72) Erfinder: Ulmer, Wolfgang, Dr.
Dr. Fuchs-Strasse 8
D-6382 Friedrichsdorf(DE)

(72) Erfinder: Wetekam, Waldemar, Dr.
Zeilring 40/1
D-6239 Eppstein/Taunus(DE)

(54) **Biologisch aktive Derivate des Human-gamma-Interferons, ihre Herstellung und Arzneimittel, die solche Derivate enthalten.**

(57) Proteine mit Human-$\gamma$-Interferon-Aktivität werden erhalten, wenn man Proteine oder Fusionsproteine, die eine Aminosäuresequenz enthalten, die im wesentlichen der von Human-$\gamma$-Interferon entspricht, proteolytisch spaltet, wobei ein Fragment erhalten wird, das mit $^6$Pro beginnt. Diese Proteine können auch in an sich bekannter Weise gentechnisch hergestellt werden.

EP 0 219 781 A2

Biologisch aktive Derivate des Human-γ-Interferons, ihre
Herstellung und Arzneimittel, die solche Derivate enthalten

Die ursprünglich veröffentlichte Aminosäuresequenz von
Human-γ-Interferon, im folgenden IFN-γ, wies 146 Aminosäuren auf (Devos et al., Nucl. Acids Res. 10 (1982) 2487).
Nach einer späteren Veröffentlichung (Rinderknecht et al.,
J. Biol. Chem. 259 (1984) 6790) fehlen jedoch dem "reifen"
IFN-γ die ersten drei Aminosäuren aus der ursprünglich
publizierten Sequenz. Im folgenden wird jedoch bei der
Numerierung der Aminosäuren die Sequenz von Devos et al.
beibehalten.

Aus der europäischen Patentanmeldung mit der Veröffentlichungsnummer 146 944 ist es bekannt, gentechnisch Derivate
des IFN-γ herzustellen, bei denen in Position 9 Lys durch
Gln ersetzt ist und zusätzlich weitere Modifikationen durchgeführt sind. Unter diesen Modifikationen sind auch N-terminale Verkürzungen um 4 bzw. 7 Aminosäuren, die durch
Bal 31-Verdauung der DNA und Expression gewonnen wurden.
Diese Derivate des IFN-γ sollen eine dem natürlichen IFN-γ
vergleichbare oder sogar erhöhte Aktivität besitzen.

Es wurde auch schon vorgeschlagen (nicht vorveröffentlichte
deutsche Offenlegungsschrift 3 414 831), Derivate des IFN-γ
herzustellen, bei denen die ersten vier Aminosäuren der
natürlichen Sequenz entfallen können, ohne daß die
biologische Aktivität des IFN-γ nennenswert beeinträchtigt
wäre und/oder die nach der Aminosäure 127 (Alanin)
folgenden Aminosäuren eliminiert werden können, wobei
ebenfalls biologisch aktive Produkte erhalten werden.

Es wurde nun gefunden, daß auch nach Abspaltung der Aminosäure in Position 5 (Asp) der natürlichen Sequenz ein
biologisch aktives IFN-γ erhalten wird. Die Erfindung betrifft somit Teilsequenzen des IFN-γ, deren Aminosäuresequenz mit der Position 6 (Pro) beginnt und mindestens
bis zur Position 127 (Ala) reicht. Weitere Aspekte der Erfindung und ihre bevorzugten Ausgestaltungen sind im folgenden bzw. in den Patentansprüchen wiedergegeben.

Es wurde weiterhin gefunden, daß man die erfindungsgemäßen
verkürzten IFN-γ-Teilsequenzen besonders vorteilhaft durch
Säurespaltung der Asp-Pro-Bindung (Piszkiewicz et al.,
Biochemical and Biophysical Research Communications 40
(1970) 1173) und Renaturierung herstellen kann. Es ist
überraschend, daß unter diesen drastischen Bedingungen ein
biologisch aktives IFN-γ-Derivat erhalten wird, da IFN-γ
als säurelabil gilt (z.B. europäische Patentanmeldung
88 540, Seite 5, Zeilen 33 - 36).

Als Ausgangsmaterial für dieses Verfahren dienen alle
Polypeptide, die entsprechend dem natürlichen IFN-γ in
Position 5 Asparaginsäure und in Position 6 Prolin enthalten. Vorteilhaft führt man diese Spaltung mit Fusionsproteinen durch, die N-terminal vor der Asparaginsäure und
gegebenenfalls vor weiteren Aminosäuren der natürlichen
oder einer abgewandelten IFN-γ-Sequenz eine mehr oder
weniger lange Aminosäuresequenz eines beispielsweise bakteriellen Proteins gebunden enthalten.

Diese Säurespaltung von Polypeptiden mit der Aminosäurefolge Asp-Pro ist nicht auf natürliches IFN-γ beschränkt,
sondern grundsätzlich für alle Polypeptide mit im wesentlichen IFN-γ-Aminosäurefolge brauchbar, die hinreichend
säurestabil sind, so daß die erhaltenen Spaltprodukte -
oder gegebenenfalls nur das erwünschte Spaltprodukt - nicht
oder nicht irreversibel verändert werden.

Die Verfahrensbedingungen richten sich naturgemäß nach
der Säurestabilität der Spaltprodukte bzw. des erwünschten
Spaltprodukts und sind zweckmäßig durch einfache Vorversuche zu ermitteln. Als Säuren verwendet man vorteilhaft
Ameisensäure, Salzsäure oder andere vergleichbare, nichtoxidierende Säuren wie verdünnte Schwefelsäure, Phosphorsäure, halogenierte Essigsäuren oder Sulfonsäuren.

Da die Reaktion im wäßrigen Medium erfolgt, sind grundsätzlich Temperaturen von etwa 0 bis etwa 100°C möglich. Bei niedrigen Temperaturen verläuft das Verfahren schonender, benötigt aber mehr Zeit. Temperaturen in der Nähe des Siedepunktes erfordern zwar weniger Zeit, sind aber nur für sehr stabile Produkte brauchbar. Bevorzugt wird deshalb ein Temperaturbereich von 10 bis 70, insbesondere von 20 bis 60°C. Die Säurekonzentration im wäßrigen Medium richtet sich nach der Stärke der Säure und der Empfindlichkeit des Produktes. Vorteilhaft verwendet man Ameisensäure in einem Konzentrationsbereich von 70 bis 80 Gew.-%, bezogen auf das wäßrige Medium.

Das Spaltungsverfahren kann in homogener oder heterogener Phase durchgeführt werden. Relativ niedermolekulare Proteine werden zweckmäßig in der wäßrigen Säurelösung gelöst oder zunächst suspendiert bzw. teilweise gelöst, worauf dann im Laufe des Verfahrens eine mehr oder weniger vollständige Auflösung eintritt.

Nach Abschluß der spezifischen Säurespaltung wird das Reaktionsgemisch zweckmäßig neutralisiert, vorteilhaft unter Kühlung, und das gewünschte Produkt nach an sich bekannten Methoden abgetrennt und renaturiert. Man kann beispielsweise ein geeignetes Adsorptionsmittel dem Gemisch zusetzen und aus dem beladenen Adsorbens das gewünschte Produkt eluieren. Zusätzlich oder an Stelle der vorstehend beschriebenen Maßnahme kann auch das Produkt durch eine oder mehrere Fällungsoperationen gereinigt werden. Es ist auch möglich, das gewünschte Polypeptid mit Hilfe einer mit spezifischen Antikörpern beladenen Säule zu isolieren.

Die Herstellung der erfindungsgemäßen verkürzten IFN-γ-Teilsequenzen und der biologisch aktiven Analoga kann vorteilhaft durch gentechnische Verfahren erfolgen, bei denen in einem Wirtsorganismus, vorzugsweise E. coli, ein Gen exprimiert wird, das für die erfindungsgemäßen Aminosäuresequenzen codiert. Diese Gene sind nach an sich bekannten Verfahren synthetisierbar oder durch Abwandlung von Genen erhältlich, wie sie beispielsweise in den europäischen Patentanmeldungen mit den Publikationsnummern 77 670, 88 540, 89 676, 95 350, 146 354, 146 944 oder 155 590 beschrieben sind bzw. in der deutschen Offenlegungsschrift 3 414 831 vorgeschlagen wurden.

Wenn die gentechnische Synthese der erfindungsgemäßen IFN-γ-Derivate nach dem Verfahren der deutschen Offenlegungsschrift 3 409 966 bzw. der europäischen Patentanmeldung mit der Veröffentlichungsnummer 155 590 erfolgt, entfallen in dem synthetischen Gen die Codons für die ersten fünf Aminosäuren der IFN-γ-Sequenz. Bei der Synthese des Gens können also beispielsweise an Stelle der Bausteine Ia bis Id in der DNA-Sequenz II die folgenden Oligonucleotide eingesetzt werden:

|  |  |  | Met | Pro | Tyr | Val | Lys | Glu | Ala |
|---|---|---|---|---|---|---|---|---|---|
| 5' | AA | TTC | ATG | CCG | TAC | GTT | AAA | GAA | GCT |
| 3' (Eco RI) | G | TAC | GGC | ATG | CAA | TTT | CTT | CGA |  |

|  | Glu | Asn | (Leu) | (Lys) |  |
|---|---|---|---|---|---|
| 5' | GAA | AAC |  |  | 3' |
| 3' | CTT | TTG | GAC | TTT | 5' |

Möglich ist auch die gentechnische Synthese nach dem in deutschen Offenlegungsschrift 3 414 831 (europäische Patentanmeldung mit der Veröffentlichungsnummer 161 504) vorgeschlagenen Verfahren. In dieser Anmeldung sind auch mehrere Variationen modifizierter DNA-Sequenzen aufgeführt, die zu IFN-γ-Analogen mit veränderter Aminosäuresequenz führen.

Die synthetischen Gene können natürlich auch in Genkonstruktionen eingebaut werden, die bei der Expression zu Fusionsproteinen führen. Solche Verfahren sind in großer Zahl bekannt. Hier soll nur beispielhaft auf die europäischen Patentanmeldungen mit den Veröffentlichungsnummern 1930 und 12494 verwiesen werden, in denen Fusionsproteine mit einem ß-Galactosidaseanteil beschrieben sind. Man kann so unlösliche Fusionsproteine gewinnen, die leicht von den anderen Proteinen abzutrennen und zu reinigen sind. Diese Fusionsproteine werden dann zweckmäßig der vorstehend beschriebenen Proteolyse unterworfen.

In den folgenden Beispielen wird die Erfindung näher erläutert. Prozentangaben beziehen sich hierbei auf das Gewicht, wenn nichts anderes angegeben ist.

<u>Beispiel 1</u>

(Direktexpression)

10 µg des Plasmids gemäß Figur 5 der deutschen Offenlegungsschrift 3 409 966, das das IFN-γ-Gen enthält, werden mit den Restriktionsendonucleasen Bam HI und Sal I geschnitten. Auf einem 2 %igen niedrigschmelzenden Agarosegel wird anschließend ein etwa 340 bp umfassendes Genfragment vom Restplasmid abgetrennt und (nach Maßgabe des Herstellers der Agarose) isoliert. Das isolierte Genfragment entspricht den in der Deutschen Offenlegungsschrift 3 409 966 beschriebenen DNA-Sequenzen IFN-I und IFN-II aus der DNA-Sequenz II (Aminosäuren 35-146).

An dieses Genfragment wird nun ein neu kombiniertes Genfragment ligiert, das seinerseits durch Ligationsreaktion der DNA-Sequenz

|  |  |  | Met | Pro | Tyr | Val | Lys | Glu | Ala |
|---|---|---|---|---|---|---|---|---|---|
| 5' | AA | TTC | ATG | CCG | TAC | GTT | AAA | GAA | GCT |
| 3' (Eco RI) | | G | TAC | GGC | ATG | CAA | TTT | CTT | CGA |

| | Glu | Asn | (Leu) | (Lys) |
|---|---|---|---|---|
| 5' | GAA | AAC | | 3' |
| 3' | CTT | TTG | GAC | TTT | 5' |

und den Oligonucleotiden Ig bis Ij der DNA-Sequenz II (IFN-I) der deutschen Offenlegungsschrift 3 409 966 erhalten wird. Anschließend wird das so erhaltene um die ersten fünf Aminosäuren verkürzte IFN-γ-Gen mit den Restriktionsenzymen Eco RI und Sal I nachgeschnitten und das 414 bp umfassende Gen durch Gelelektrophorese aus einem 2 %igen Agarosegel abgetrennt.

Das Expressionsplasmid gemäß Figur 5 der deutschen Offenlegungsschrift 3 409 966 wird mit den Restriktionsenzymen Eco RI und Sal I geöffnet, das darin enthaltene IFN-γ-Gen in einem 0,7 %igen Agarosegel abgetrennt und das Restplasmid mit dem neu konstruierten verkürzten IFN-γ-Gen mit Hilfe des Enzyms T4 DNA-Ligase ligiert. Transformation, Anzucht und Induktion des verkürzten IFN-γ-Gens erfolgen in bekannter Weise, beispielsweise gemäß deutscher Offenlegungsschrift 3 409 966 bzw. nach dem in der deutschen Offenlegungsschrift 3 414 831 vorgeschlagenen Verfahren. Man erhält ein Polypeptid mit der Aminosäuresequenz 6-146 von IFN-γ, N-terminal verlängert um Met.

Beispiel 2

a) Herstellung eines Fusionsproteins

Man geht aus von dem synthetischen Gen gemäß der deutschen Offenlegungsschrift 3 409 966 (europäische Anmeldung mit der Veröffentlichungsnummer 155 590), das wie folgt an das ß-Galactosidasegen gekoppelt wird:

Das Plasmid gemäß Figur 5 dieser Offenlegungsschrift (oder das nach Beispiel 1 erhaltene analoge Plasmid mit der verkürzten IFN-γ-Sequenz) wird mit Sal I aufgeschnitten und mit dem folgenden Sal I-Eco RI-Adaptor ligiert:

```
5' TCG ACC CGG GCT G        3'
3'        GG GCC CGA CTT AA 5'
   (Sal I)           (Eco RI)
```

Aus dem Ligierungsprodukt wird mit Eco RI das verlängerte Gen, das jetzt an beiden Enden von Eco RI-Erkennungssequenzen flankiert ist, abgetrennt, über ein 2 %iges niedrigschmelzendes Agarosegel gereinigt und isoliert.

Das Plasmid pBH 20 (E.L. Winnacker, Gene und Klone, eine Einführung in die Gentechnologie, VCH Verlagsgesellschaft, Weinheim (1984), S. 233 - 234; europäische Patentanmeldung 12 494, Fig. 1) wird mit dem Restriktionsenzym Eco RI geöffnet und das Gen mit der IFN-γ-Sequenz in das linearisierte Plasmid eingesetzt. Nach Transformation und Induktion wird ein Fusionsprotein gebildet, das im Anschluß an die ersten etwa 1000 Aminosäuren der ß-Galactosidase die (komplette bzw. verkürzte) Aminosäuresequenz des IFN-γ enthält.

b) Proteolyse des Fusionsproteins

1 g des angereicherten Fusionsproteins wird in 100 ml 70 %iger Ameisensäure gelöst und 6 Stunden bei 60°C gerührt. Nach Ablauf der Reaktionszeit wird die Lösung mit 4 N Natronlauge unter Kühlung neutralisiert und mit Wasser auf das 5-fache bis 10-fache Volumen verdünnt. Zur Adsorption des gewünschten Spaltprodukts werden 100 g Kieselsäure eingerührt. Die Adsorption erfolgt während 3 Stunden unter Rühren bei 8°C. Anschließend wird die beladene Kieselsäure in eine Chromatographiesäule gefüllt und bis zur Konstanz der Extinktion (278 nm) mit 50 mM Phosphatpuffer; 125 mM Natriumchlorid (pH 7,4) gewaschen. Die Elution des Interferons mit der Aminosäuresequenz 6-146 des IFN-γ erfolgt mit 7 M Guanidin; 0,1 % Tensid (auf Basis Polyoxyethylensorbitan-Monolaurat); 0,1 M Tris-HCl (pH 7,4). Zur Renaturierung wird das Eluat mit 0,1 M Tris-HCl (pH 7,4) auf das 10-fache Volumen verdünnt und 3 - 4 Stunden bei 20°C gerührt. Nach Dialyse über eine Membran mit der Ausschlußgrenze 10 000 - 12 000 Dalton, 2 x gegen je 10 l 0,6 M Ammoniumhydrogencarbonat; 10 mM Magnesiumsulfat (pH 8) und 1 x gegen 10 l 0,6 M Ammoniumhydrogencarbonat (pH 8), wird die IFN-γ-Lösung mit 1,5 g Serumalbumin versetzt und gefriergetrocknet. Man erhält 2,1 g Lyophilisat, in dem mittels Radioimmunoassay 205 x $10^7$ Einheiten IFN-γ bestimmt werden ($\hat{=}$ ca. 40 mg).

Patentansprüche:

1. Proteine mit einer Aminosäuresequenz, die im wesentlichen der des Human-γ-Interferons von Aminosäure 6 (Pro) bis mindestens 127 (Ala) entspricht.

2. Proteine mit Human-γ-Interferon-Teilsequenzen, die N-terminal mit $^6$Pro beginnen und C-terminal mindestens bis zu $^{127}$Ala reichen.

3. Human-γ-Interferon-Teilsequenz, bestehend aus der Aminosäuresequenz 6 (Pro) bis 146 (Gln).

4. Verfahren zur Herstellung von Proteinen mit einer Aminosäuresequenz, die im wesentlichen der des Human-γ-Interferons entspricht, jedoch N-terminal bis einschließlich $^5$Asp verkürzt ist, dadurch gekennzeichnet, daß man ein Protein oder Fusionsprotein, das im wesentlichen die Aminosäurefolge von Human-γ-Interferon enthält, proteolytisch spaltet und das N-terminal mit Prolin beginnende Polypeptid isoliert und renaturiert.

5. Verfahren zur gentechnischen Herstellung der Proteine nach Anspruch 1 bis 3, dadurch gekennzeichnet, daß man in einer Wirtszelle ein Gen zur Expression bringt, das für die Aminosäuresequenz des gewünschten Proteins kodiert.

6. Arzneimittel mit Human-γ-Interferonwirkung, gekennzeichnet durch einen Gehalt an einem Protein gemäß Anspruch 1, 2 oder 3.

7. Verwendung eines Proteins gemäß Anspruch 1, 2 oder 3 zur Herstellung eines Arzneimittels mit Human-γ-Interferonwirkung.

Patentansprüche Österreich, Spanien und Griechenland:

1. Verfahren zur Herstellung von Proteinen mit einer Aminosäuresequenz, die im wesentlichen der des Human-$\gamma$-Interferons entspricht, jedoch N-terminal bis einschließlich $^5$Asp verkürzt ist, dadurch gekennzeichnet, daß man ein Protein oder Fusionsprotein, das im wesentlichen die Aminosäurefolge von Human-$\gamma$-Interferon enthält, proteolytisch spaltet und das N-terminal mit Prolin beginnende Polypeptid isoliert und renaturiert.

2. Verfahren nach Anspruch 1, dadurch gekennzeichnet, daß das zu spaltende Protein im Anschluß an $^6$Pro die Aminosäuresequenz des Human-$\gamma$-Interferons bis $^{146}$Gln hat.

3. Verfahren nach Anspruch 1 oder 2, dadurch gekennzeichnet, daß die Spaltung bei 10 bis 70°C erfolgt.

4. Verfahren nach Anspruch 1, 2 oder 3, dadurch gekennzeichnet, daß die Spaltung bei 20 bis 60°C erfolgt.

5. Verfahren zur gentechnischen Herstellung der in Anspruch 1 oder 2 definierten Proteine, dadurch gekennzeichnet, daß man in einer Wirtszelle ein Gen zur Expression bringt, das für die Aminosäuresequenz des gewünschten Proteins kodiert.